# EUROPEAN PATENT APPLICATION

(11) **EP 4 553 151 A1**
(43) Date of publication of application: **14.05.2025**
(21) Application number: 23924256.3
(22) Date of filing: 26.12.2023
(51) Int. Cl.: C12N 5/0775, C12N 5/077

(54) **METHOD FOR CULTURING MESENCHYMAL STEM CELLS**

(30) Priority: 21.02.2023 JP 2023025297
(71) Applicant: Pola Chemical Industries, Inc., Shizuoka 437-8765 (JP)
(72) Inventor: KUGE, Takayuki, Yokohama-shi, Kanagawa 244-0812 (JP); YOSHIZAWA, Misa, Yokohama-shi, Kanagawa 244-0812 (JP); IWANAGA, Tomoyuki, Yokohama-shi, Kanagawa 244-0812 (JP)
(74) Representative: Witthoff Jaekel Steinecke Patentanwälte PartG mbB
(86) International application number: PCT/JP2023/046567
(87) International publication number: WO 2024/176612

(57) **Abstract**

An object of the present invention is to provide a novel technique for easy culture of mesenchymal stem cells from a removed hair.

The present invention to solve the above problem is a method of culturing mesenchymal stem cells, and the method includes a culture step of culturing a hair acquired by removal and including a hair bulb covered with a dermal sheath tissue in a culture solution.

## Description

### Technical Field

The present invention relates to a method of culturing mesenchymal stem cells.

### Background Art

Mesenchymal stem cells (MSCs) are also called mesenchymal stromal cells, and are one kind of somatic stem cells present in bone marrow, an adipose tissue, a placenta, an umbilical cord, an egg membrane, a hair follicle, and the like. Mesenchymal stem cells are pluripotent cells having a potential for differentiation into a bone, a cartilage, and adipocytes, and have a low risk of canceration. Therefore, mesenchymal stem cells have been studied as a source of cellular medicine, regenerative medicine, and the like to replace induced pluripotent stem cells.

Mesenchymal stem cells are generally acquired from a tissue with an invasive method. For example, bone marrow MSCs (BM-MSCs) are acquired from an iliac crest by femoral puncture, and adipose MSCs (AD-MSCs) are acquired from subcutaneous fat by liposuction and lipectomy. Dermal papilla cells, which are mesenchymal stem cells of a hair follicle, are acquired from an excised skin tissue.

Mesenchymal stem cells can also be separated and cultured using a sample acquired with a non-invasive method. For example, Patent Literature 1 discloses a method of separating and culturing mesenchymal stem cells by excising a hair bulb part from a removed hair and culturing the obtained hair in the bulge region on a Transwell membrane.

### Citation List

### Patent Literature

Patent Literature 1: WO 2021/009246 A

### Summary of Invention

### Technical Problem

Considering the above-described prior technique, an object of the present invention is to provide a novel technique for easy culture of mesenchymal stem cells from a removed hair.

### Solution to Problem

The present invention that solves the above problem is a method of culturing mesenchymal stem cells, and the method includes a culture step of culturing a hair acquired by removal and including a hair bulb covered with a dermal sheath tissue in a culture solution.

According to the present invention, mesenchymal stem cells can be acquired by using a hair bulb portion of a removed hair. The culture sample can be easily acquired by hair pulling, which is a non-invasive method, and thus the burden on the donor individual at the time of acquiring the sample can be reduced.

In a preferred mode of the present invention, the mesenchymal stem cells are derived from the hair bulb.

The mesenchymal stem cells can be efficiently obtained by proliferation of mesenchymal stem cells from the vicinity of the hair bulb.

In a preferred mode of the present invention, the culture step includes a static culture step of static-culturing the hair while entire replacement of a medium is not performed and the medium is replenished with a fresh medium.

If the static culture step is included, the proliferation of mesenchymal stem cells from the hair can be promoted.

In a preferred mode of the present invention, a culture period in the static culture step is 6 days or more from a start of the static-culturing.

If the static culture period is set within the above range, the proliferation of mesenchymal stem cells after the static-culturing can be promoted.

In a preferred mode of the present invention, the method of culturing includes a step of bringing the hair that is acquired into contact with an antiseptic solution, before the culture step, for less than 1 minute before the culturing.

If the above step is included, the adhesion of the sample hair to a culture vessel material is improved, and the mesenchymal stem cells can be efficiently acquired from the vicinity of the hair bulb of the hair.

In a preferred mode of the present invention, the method of culturing includes a step of seeding one of the hair in one well in a culture vessel before the culture step.

The present invention including the above step enables proliferation from one hair to a sufficient amount of mesenchymal stem cells.

In a preferred mode of the present invention, the method of culturing does not include a decomposition step of decomposing a constituent component of an extracellular matrix in the hair before the culture step.

In a mode that does not include the decomposition step, damage to cells can be reduced, and the mesenchymal stem cells can be efficiently acquired from the vicinity of the hair bulb of the hair. Furthermore, treatment of a sample is easier and the step can be simpler than in a conventional method including the decomposition step.

In a preferred mode of the present invention, the decomposition step is a step of treating the hair with a collagenase.

If the collagenase treatment is not performed before the culture step, the mesenchymal stem cells can be efficiently acquired from the vicinity of the hair bulb of the hair, and the step can be further simpler.

In a preferred mode of the present invention, the mesenchymal stem cells are subjected to adherent culture in the culture step.

In a preferred mode of the present invention, the adherent culture is performed on a culture surface coated with laminin or a fragment of laminin.

In the above mode, the proliferation of mesenchymal stem cells can be efficiently performed.

In a preferred mode of the present invention, the culture solution contains L-ascorbic acid, selenium, transferrin, and insulin.

The proliferation of mesenchymal stem cells can be efficiently performed by using the above culture solution.

In a preferred mode of the present invention, the culture solution contains a basic fibroblast growth factor (bFGF) and/or an epidermal growth factor (EGF).

The proliferation of mesenchymal stem cells can be efficiently performed by using the above culture solution.

Furthermore, the present invention relates to a method of producing mesenchymal stem cells, and this method includes the method of culturing mesenchymal stem cells.

The present invention also relates to mesenchymal stem cells that are acquired with the method of culturing mesenchymal stem cells. In a preferred mode of the present invention, the mesenchymal stem cells have a potential for differentiation into one kind or two or more kinds of cells selected from osteoblasts, adipocytes, and chondrocytes.

Furthermore, the present invention relates to a method of producing differentiated cells, and the method includes the steps of obtaining a cell population containing mesenchymal stem cells with the method of culturing mesenchymal cells, and culturing the cell population containing the mesenchymal stem cells in a differentiation induction medium to obtain a cell population containing one kind or two or more kinds of differentiated cells selected from osteoblasts, adipocytes, and chondrocytes.

The present invention also relates to differentiated cells that are produced with the method of producing differentiated cells.

The present invention to solve the above problem is as follows.
[1] A method of culturing mesenchymal stem cells, the method including a culture step of culturing a hair acquired by removal and including a hair bulb covered with a dermal sheath tissue in a culture solution.
[2] The method of culturing according to [1], wherein the mesenchymal stem cells are derived from the hair bulb.
[3] The method of culturing according to [1] or [2], wherein the culture step includes a static culture step of static-culturing the hair while entire replacement of a medium is not performed and the medium is replenished with a fresh medium.
[4] The method of culturing according to [3], wherein a culture period in the static culture step is 6 days or more from a start of the static-culturing.
[5] The method of culturing according to any one of [1] to [4], including a step of bringing the hair that is acquired into contact with an antiseptic solution, before the culture step, for less than 1 minute before the culturing.
[6] The method of culturing according to any one of [1] to [5], including a step of seeding one of the hair in one well in a culture vessel before the culture step.
[7] The method of culturing according to any one of [1] to [6], not including a decomposition step of decomposing a constituent component of an extracellular matrix in the hair before the culture step.
[8] The method of culturing according to [7], wherein the decomposition step is a step of treating the hair with a collagenase.
[9] The method of culturing according to any one of [1] to [8], wherein the mesenchymal stem cells are subjected to adherent culture in the culture step.
[10] The method of culturing according to [9], wherein the adherent culture is performed on a culture surface coated with laminin or a fragment of laminin.
[11] The method of culturing according to any one of [1] to [10], wherein the culture solution contains L-ascorbic acid, selenium, transferrin, and insulin.
[12] The method of culturing according to any one of [1] to [11], wherein the culture solution contains a basic fibroblast growth factor (bFGF) and/or an epidermal growth factor (EGF).
[13] A method of producing mesenchymal stem cells, the method including the method of culturing according to any one of [1] to [12].
[14] Mesenchymal stem cells that are acquired with the method of culturing according to any one of [1] to [12].
[15] The mesenchymal stem cells according to [14], having a potential for differentiation into one kind or two or more kinds of cells selected from osteoblasts, adipocytes, and chondrocytes.
[16] A method of producing differentiated cells, the method including the steps of:
   obtaining a cell population containing mesenchymal stem cells with the method of culturing according to any one of [1] to [12]; and
   culturing the cell population containing the mesenchymal stem cells in a differentiation induction medium to obtain a cell population containing one kind or two or more kinds of differentiated cells selected from osteoblasts, adipocytes, and chondrocytes.
[17] Differentiated cells that are produced with the method of producing according to [16].

### Advantageous Effects of Invention

According to the present invention, mesenchymal stem cells can be efficiently acquired from a hair acquired from any individual. Furthermore, differentiated cells such as adipocytes can be produced using the mesenchymal stem cells.

### Brief Description of Drawings

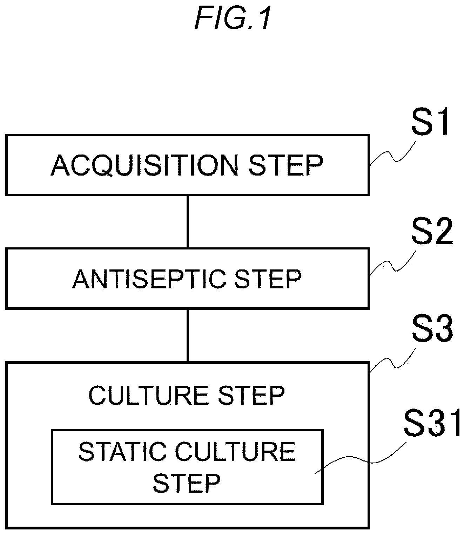

Fig. 1 is a step diagram for explanation of steps of a method of culturing mesenchymal stem cells according to an embodiment of the present invention.

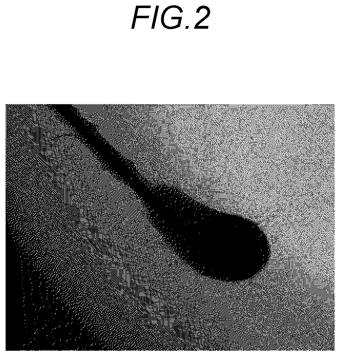

Fig. 2 is a bright field photograph showing a state of mesenchymal stem cells obtained by proliferation from the vicinity of a hair bulb of a removed hair in Example 1.

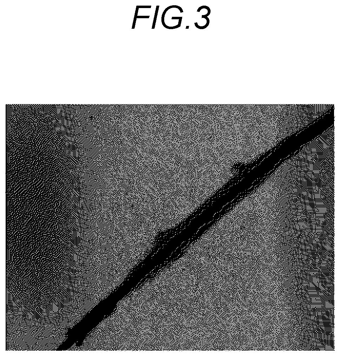

Fig. 3 is a bright field photograph showing a state of keratinocytes obtained by proliferation from the vicinity of an outer root sheath adhered to a removed hair in Example 1.

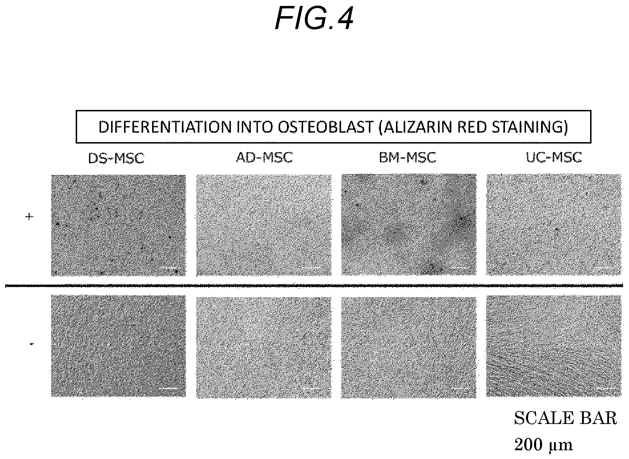

Fig. 4 shows bright field photographs showing the results of alizarin red staining performed after culturing hair-derived mesenchymal stem cells (DS-MSCs) cultured in Example 1, adipose tissue-derived mesenchymal stem cells (AD-MSCs), bone marrow-derived mesenchymal stem cells (BM-MSCs), and umbilical cord-derived mesenchymal stem cells (UC-MSCs) in an osteoblast induction medium in Example 2. "+" indicates that the culture was performed in a differentiation induction medium, and "-" indicates that the culture was performed in an undifferentiation maintenance medium.

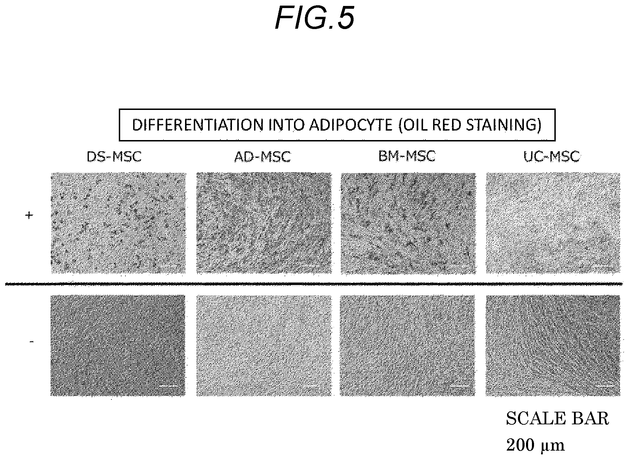

Fig. 5 shows bright field photographs showing the results of oil red staining performed after culturing hair-derived mesenchymal stem cells (DS-MSCs) cultured in Example 1, adipose tissue-derived mesenchymal stem cells (AD-MSCs), bone marrow-derived mesenchymal stem cells (BM-MSCs), and umbilical cord-derived mesenchymal stem cells (UC-MSCs) in an adipocyte induction medium in Example 2. "+" indicates that the culture was performed in a differentiation induction medium, and "-" indicates that the culture was performed in an undifferentiation maintenance medium.

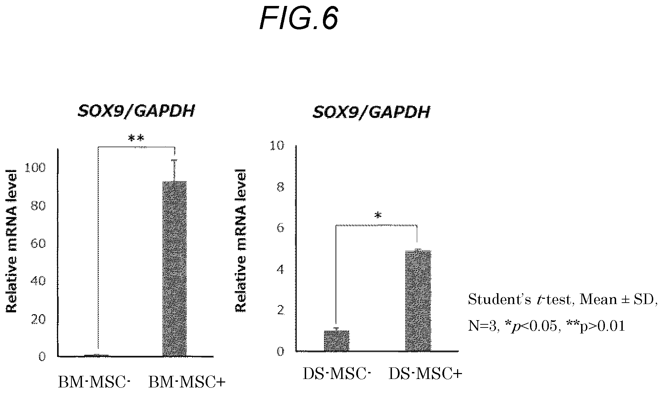

Fig. 6 shows graphs showing the results of analyzing the relative expression level of a chondrocyte marker gene (SOX9) with respect to the expression level of GAPDH, which is a housekeeping gene, after culturing hair-derived mesenchymal stem cells (DS-MSCs) cultured in Example 1 and bone marrow-derived mesenchymal stem cells (BM-MSCs) in a chondrocyte induction medium in Example 2. Cells cultured in a differentiation induction medium are denoted by "+", cells cultured in an undifferentiation maintenance medium are denoted by "-", and the expression level of cells cultured in an undifferentiation maintenance medium is used as a reference (1).

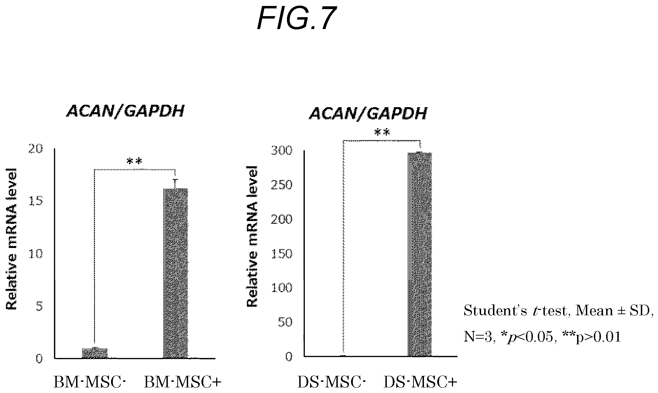

Fig. 7 shows graphs showing the results of analyzing the relative expression level of a chondrocyte marker gene (ACAN) with respect to the expression level of GAPDH, which is a housekeeping gene, after culturing hair-derived mesenchymal stem cells (DS-MSCs) cultured in Example 1 and bone marrow-derived mesenchymal stem cells (BM-MSCs) in a chondrocyte induction medium in Example 2. Cells cultured in a differentiation induction medium are denoted by "+", cells cultured in an undifferentiation maintenance medium are denoted by "-", and the expression level of cells cultured in an undifferentiation maintenance medium is used as a reference (1).

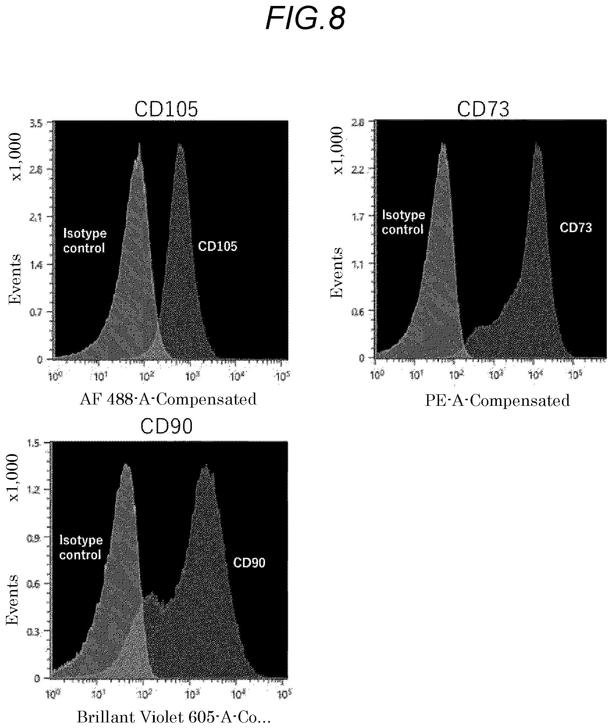

Fig. 8 shows graphs showing the results of analyzing a mesenchymal stem cell positive marker (CD105, CD73, or CD90) of hair-derived mesenchymal stem cells (DS-MSCs) cultured in Example 1 by fluorescence-activated cell sorting (FACS) in Example 3.

### Description of Embodiments

### <Method of Culturing Mesenchymal Stem Cells>

Subsequently, a preferred embodiment of the method of culturing mesenchymal stem cells of the present invention will be described with reference to Fig. 1.

Note that the present invention is not limited to the present embodiment, and can be appropriately modified in design.

The method according to Fig. 1 includes an acquisition step S1 of acquiring a hair including a hair bulb covered with a dermal sheath tissue from any individual, an antiseptic step S2 of disinfecting the obtained hair, and a culture step S3 of culturing the hair.

Here, the term "hair bulb" in the present invention refers to a bulged portion of a hair, and the portion is positioned in an inner deep part of the hair in the skin. At the center of the hair bulb, a dermal papilla is present. Dermal papilla cells constituting the dermal papilla are known as a kind of mesenchymal stem cells.

The term "dermal sheath (DS) tissue" in the present invention refers to a single layer or several layers of dermal tissue enveloping the outermost layer of a hair follicle, and the tissue is configured to surround an epithelial outer root sheath. The dermal sheath is connected to the dermal papilla at the lowermost end of the hair bulb part.

Hereinafter, details of each of the acquisition step S1, the antiseptic step S2, and the culture step S3 according to the present invention will be described.

### (1) Acquisition Step S1

The hair used in the present invention is acquired by removal from any individual, preferably a mammal (for example, human, mouse, monkey, cattle, pig, rat, or dog). In the present invention, the hair is preferably acquired from a human.

If the hair is acquired by removal, a sample can be acquired in which a dermal sheath tissue is adhered to a hair bulb portion. Because a hair available by the non-invasive method is used, the burden can be reduced at the time of collecting the sample from the donor individual.

The individual (specifically, mammalian individual) from which a tissue for acquisition of the hair is collected is not particularly limited. For example, in the case of using mesenchymal stem cells as a source of cells for screening for evaluation of a cosmetic effect, drug sensitivity, the presence or absence of a side effect, and the like in the donor individual, the hair is preferably acquired from the donor or another person having the same genetic polymorphism correlated with drug sensitivity and the like as the donor.

In the case of use, for example, for transplantation into a patient, the hair is preferably acquired from the donor such as a patient, or from another person having the same or substantially the same HLA type as the donor. Thus, the risk of rejection is avoided, and therefore the obtained mesenchymal stem cells can be suitably used for regenerative medicine.

Here, the term "another person having substantially the same HLA type" means that the HLA types of the donor and another person match to such an extent that when the obtained mesenchymal stem cells are transplanted to the donor individual by using an immunosuppressive agent or the like, the transplanted cells can engraft. Examples of such a case include a case where HLA (for example, three gene loci of HLA-A, HLA-B, and HLA-DR) is identical.

The number of hairs removed in the present invention is to be at least one, and is preferably two or more, and more preferably five or more. Specifically, 1 to 10 hairs are preferably used. Then, the lower part of the hair collected from the donor individual is cut out to acquire a hair bulb part covered with a dermal sheath tissue. The hair including the hair bulb to be acquired preferably has a length of 0.3 to 5 mm.

The present invention may include a step of excising a region to which an outer root sheath of the hair is adhered to acquire only the hair bulb part.

If this step is included, only mesenchymal stem cells can be efficiently acquired that proliferate from the vicinity of the hair bulb.

Although the mode including the acquisition step S1 of acquiring a hair from a donor individual is exemplified, in the present invention, a mode is also preferable in which a hair acquired in advance by removal is optionally subjected to the following antiseptic step S2 and then the culture step S3 is performed.

### (2) Antiseptic step S2

In the antiseptic step S2, the acquired hair is brought into contact with an antiseptic solution before the culture step S3. The method of bringing the hair into contact with the antiseptic solution is not particularly limited, and immersing the hair in the antiseptic solution is preferable.

The contact time with the antiseptic solution is preferably less than 1 minute, more preferably less than 30 seconds, still more preferably less than 10 seconds, and particularly preferably less than 5 seconds.

If the contact time with the antiseptic solution is set within the above range, damage to the hair is reduced, the adhesion of the hair to a culture vessel material is improved, and thus proliferation of the mesenchymal stem cells can be efficiently performed.

The antiseptic solution used in the present invention is preferably a solution to which an antibiotic is added. One kind or two or more kinds of antibiotics are preferably used that are selected from cell wall synthesis inhibitors, protein synthesis inhibitors, and cell membrane function inhibitors. More preferably, one or more kinds of antibiotics are selected from each of cell wall synthesis inhibitors, protein synthesis inhibitors, and cell membrane function inhibitors.

Preferred examples of cell wall synthesis inhibitors include β-lactam antibiotics. Examples of β-lactam-based cell wall synthesis inhibitors include penicillin, ampicillin, and carbenicillin sodium, and in the present invention, penicillin is preferably used.

Preferred examples of protein synthesis inhibitors include aminoglycoside antibiotics.

Examples of aminoglycoside protein synthesis inhibitors include kanamycin, gentamicin, streptomycin, neomycin, hygromycin, and tobramycin, and in the present invention, streptomycin is preferably used.

Preferred examples of cell membrane function inhibitors include polyene antibiotics. Examples of polyene cell membrane function inhibitors include amphotericin B, nystatin, and natamycin, and in the present invention, amphotericin B is particularly preferably used.

The antiseptic solution according to the present invention is preferably a solution containing penicillin, streptomycin, and amphotericin B as antibiotics.

### (3) Culture Step S3

After the antiseptic step S2 is performed, the culture step S3 is performed in which the acquired hair is cultured in a culture solution.

Thus, mesenchymal stem cells are acquired from the hair bulb portion of the hair.

As the culture solution used for culture of the hair, a known medium used for culturing mesenchymal stem cells can be used, and suitable examples of the medium include a DMEM medium, a DMEM/F12 medium, Essential 8, AmnioMAX (trade mark) C-100, and StemPro MSC SFM.

The culture solution used in the culture step S3 is preferably a medium containing L-ascorbic acid, selenium, transferrin, and insulin. Examples of such a medium include Essential 8. An Essential 8 medium contains L-ascorbic acid, selenium, insulin, NaHCO₃, transferrin, bFGF, and a TGFβ family signaling pathway agonist (TGFβ1 or Nodal) as additives in a DMEM/F12 medium.

The proliferation of mesenchymal stem cells can be efficiently performed by using such a culture solution.

The culture solution used in the culture step S3 preferably contains a basic fibroblast growth factor (bFGF) and/or an epidermal growth factor (EGF).

If the above factors are contained, mesenchymal stem cells can be efficiently acquired while differentiation of the cells is suppressed. Particularly if a basic fibroblast growth factor (bFGF) is contained, the adhesion of the mesenchymal stem cells obtained by proliferation can be improved.

In a preferred mode, a first culture solution can be used that is a medium obtained by adding 5 to 15 ng/mL, more preferably 10 ng/mL of bFGF and 5 to 15 ng/mL, more preferably 10 ng/mL of EGF to Essential 8.

The culture solution used in the culture step S3 is also preferably in a form in which a serum is contained. The serum is not particularly limited as long as it is derived from an animal, and is preferably a serum derived from a mammal. Specifically, preferred examples of the serum include a fetal bovine serum and a human serum.

The culture solution used in the culture step S3 preferably has a calcium concentration of 0.2 mM or more, more preferably 0.5 mM or more, preferably 0.8 mM or more, and more preferably 1.0 mM or more.

The culture solution used in the culture step S3 can have a calcium concentration of 4.0 mM or less, 3.5 mM or less, or 3.0 mM or less.

Specifically, the calcium concentration of the culture solution used in the culture step S3 is preferably 0.2 to 4 mM, more preferably 0.5 to 3.5 mM, and still more preferably 1.0 to 3.5 mM.

If the medium having a calcium concentration described above is used, proliferation of cells other than mesenchymal stem cells, such as keratinocytes, can be suppressed.

The hair is preferably subjected to adherent culture using a culture vessel to which cells can adhere.

As the culture vessel used for the adherent culture, an appropriate culture vessel can be appropriately selected according to the culture scale, the culture conditions, and the culture period. The culture vessel adhesive to cells is, for example, a culture vessel having a surface artificially treated for the purpose of improving the adhesion to cells, specifically, a culture vessel having a treated surface or a culture vessel whose inside is coated with a coating agent.

In the present invention, the hair is preferably cultured using a culture vessel coated with a coating agent.

Examples of the coating agent include extracellular matrixes such as laminin, entactin, collagen, gelatin, vitronectin, Synthemax (Corning Incorporated), and Matrigel, and polymers such as polylysine and polyornithine. In the present invention, an extracellular matrix is preferably used, and laminin or a fragment of laminin is preferably used.

Suitable examples of the laminin to be used include laminin α5β1γ1 (laminin 511), laminin α5β2γ1 (laminin 521), laminin α4β1γ1 (laminin 411), laminin α2β1γ1 (laminin 211), and laminin fragments thereof, and laminin 511 or laminin 521 is more preferably used. As such laminin and laminin fragments, recombinants can also be used.

The laminin or the laminin fragment preferably has an integrin binding site.

The laminin fragment more preferably forms a heterotrimer.

The type of integrin to which the laminin or the laminin fragment binds is preferably integrin α6β1, integrin α6β4, integrin α3β1, or integrin α7β1, and more preferably integrin α6β1. If laminin or a laminin fragment is used that binds to integrin α6β1, the proliferation of mesenchymal stem cells can be promoted.

Preferred examples of the laminin fragment having a binding activity to such integrin include a laminin fragment derived from at least one selected from laminin 511, laminin 521, laminin 411, and laminin 211, and the laminin fragment is more preferably derived from laminin 511 or laminin 521, and more preferably derived from laminin 511. Preferred examples of such a laminin fragment include an E8 fragment obtained by digesting laminin with elastase and a recombinant of an E8 fragment.

In the present invention, laminin 511E8 is more preferably used. As laminin 511E8, a commercially available product such as iMatrix-511 (manufactured by Nippi, Incorporated) can be used.

Examples of the specific form of the culture vessel include forms of a flask, a tissue culture flask, a culture dish (dish), a tissue culture dish, a multi-dish, a microplate, a microwell plate, a multi-plate, a multi-well plate, a chamber slide, a petri dish, a tube, a tray, a culture bag, a microcarrier, a bead, a stack plate, a spinner flask, and a roller bottle.

The culture vessel to be used is preferably a 12-well plate or a 24-well plate for cell culture, and more preferably a 24-well plate.

The hair is preferably seeded in a plate so that one hair is seeded in one well.

According to the present invention, a sufficient amount of mesenchymal stem cells can be efficiently obtained by proliferation from one hair (one sample). Therefore, there is no need to seed a plurality of hairs in one well, and thus there is an advantage that preparation is sufficient with a smaller amount of hairs than in the existing method of Patent Literature 1.

The amount of the culture solution at the time of seeding is preferably an amount such that the entire tissue of the cut-out hair is immersed and the liquid level is 1 mm or less above the entire tissue. If the medium at the time of seeding is set in an amount described above, the hair can be adhered to the plate.

In the culture step S3, the medium is replenished and the entire amount of the medium is replaced, and thus mesenchymal stem cells are proliferated. Here, in the culture step S3, static culture of the acquired hair is preferably performed first. Hereinafter, a static culture step S31 of static-culturing the hair will be described in detail.

In the static culture step S31, the seeded hair is preferably static-cultured while entire replacement of the medium is not performed and the medium is replenished with a fresh medium. The number of times of replenishment of the medium is preferably 1 to 4, more preferably 2 to 3, and still more preferably 3.

The amount of the medium replenished in the static culture step S31 is preferably an amount such that the entire cut-out hair is immersed and the liquid level is 2 mm or less, more preferably 1.5 mm or less above the entire hair.

The amount of the medium replenished is preferably 0.5 to 3 times, more preferably 1 to 3 times, and still more preferably 1 to 2 times the amount at the time of seeding the hair. In a case where the number of times of replenishment of the medium is 3 or more, the medium is preferably replenished with a larger amount of a medium in the third or more replenishment than in the first and the second replenishment. In such a case, the amount of a medium in the third or more replenishment is preferably 1.2 to 3 times, more preferably 1.5 to 2.5 times, and still more preferably 2 to 2.5 times the amount of a medium in the first and second replenishment. The amount of a medium in the second or more replenishment is preferably an amount such that the entire tissue of the cut-out hair is immersed and the liquid level is 1 to 2 mm, more preferably 1 to 1.5 mm above the entire tissue.

A specific mode of the static culture step S31 is preferably, for example, a mode in which the number of times of replenishment of the medium is 3, the medium is replenished with a medium in an amount equal (1 time) to that at the time of seeding once during 20 to 24 hours and once during 100 to 120 hours after seeding of the hair, and the medium is replenished with a medium in an amount 2 times that at the time of seeding once during 140 to 170 hours after seeding of the hair.

The culture period in the static culture step S31 is preferably 4 days or more, more preferably 6 days or more, and still more preferably 8 days or more. Note that the total culture period in the static culture step S31 refers to a period from the day on which the hair is seeded to entire replacement of the medium.

If the static culture step S31 as described above is performed, the proliferation of mesenchymal stem cells from the hair bulb part can be promoted.

In the culture step S3, it is preferable that, after the static culture step S31 is performed, replenishment and/or entire replacement of the medium be performed under the above conditions according to the proliferation level of the cells.

The replenishment and/or the entire replacement of the medium is preferably continued until 50 to 80% of the cells obtained by proliferation are confluent. The culture period by the replenishment and the replacement of the medium after the static culture step S31 is preferably 7 to 28 days, and more preferably 14 to 21 days.

In the culture step S3, passage is preferably performed before the density of cells becomes too large in the development region of the cells obtained by proliferation from the hair. For example, in the case of culture in a 24-well plate (well bottom diameter: 15 to 16 mm), a step is preferably included in which passage is performed at the stage when 50 to 80% of the cells obtained by proliferation from the hair become confluent and the cells are further cultured.

As the culture solution used for passage, a culture solution can be appropriately used such as a DMEM medium, a DMEM/F12 medium, Essential 8, AmnioMAX (trade mark) C-100 (Gibco), StemPro MSC SFM (Gibco), or StemFit For MSC (Ajinomoto Co., Inc.), and in the present invention, Essential 8 or StemFit For MSC can be suitably used. The culture solution used in the passage is preferably a culture solution containing L-ascorbic acid, selenium, transferrin, and insulin. The culture solution used in the passage preferably contains a basic fibroblast growth factor (bFGF) and/or an epidermal growth factor (EGF).

The culture solution used in the passage preferably has a calcium concentration of 0.2 mM or more, more preferably 0.5 mM or more, preferably 0.8 mM or more, and more preferably 1.0 mM or more.

Specifically, the calcium concentration of the culture solution used in the passage is preferably 0.2 to 4 mM, more preferably 0.5 to 3.5 mM, and still more preferably 1.0 to 3.5 mM.

In a case where a hair to which an outer root sheath is adhered is cultured as it is, keratinocyte-like cells generated by proliferation disappear by repeating passage using a culture solution having a calcium concentration within the above range, and thus mesenchymal stem cells can be separated and cultured.

In the present invention, the method of culturing preferably does not include a decomposition step of decomposing a constituent component of an extracellular matrix in the hair before the culture step S3.

In a conventional method, as shown in Patent Literature 1, at the time of culturing mesenchymal stem cells or the like from a hair, proliferation of the target cells is generally promoted by decomposing an extracellular matrix such as a collagen fiber adhered to a sample. Meanwhile, in the present invention, mesenchymal stem cells are proliferated from the vicinity of a hair bulb, and thus mesenchymal stem cells can be acquired without decomposing an extracellular matrix. Thus, according to the present invention, treatment of a sample is easier than in a conventional method, and mesenchymal stem cells can be efficiently acquired from a small number of samples.

In the present invention, a mode is more preferable in which a step of treating the hair with collagenase is not included before the culture step S3. If collagen is not decomposed by collagenase treatment, damage to the sample can be minimized, and mesenchymal stem cells can be efficiently proliferated from the vicinity of the hair bulb.

In the present invention, a mode can be adopted in which a step of treating the hair with collagenase and/or dispase is not included before the culture step S3.

According to the present invention, a sufficient amount of mesenchymal stem cells can be acquired from one hair acquired from a donor individual. That is, mesenchymal stem cells can be efficiently proliferated with a smaller number of samples than in the conventional culture method using hairs described in Patent Literature 1. Therefore, the burden of acquiring a hair from a donor individual can be reduced, and the time can be shortened that is required to acquire a necessary amount of mesenchymal stem cells.

The present invention is also a method of producing mesenchymal stem cells, including the method of culturing mesenchymal stem cells. The present invention also relates to mesenchymal stem cells that are acquired with the method of culturing mesenchymal stem cells.

The mesenchymal stem cells according to the present invention are preferably positive for 1 or more, more preferably 2 or more, and still more preferably 3 or more mesenchymal stem cell markers selected from CD105, CD73, CD90, and CD44.

Furthermore, the mesenchymal stem cells are preferably positive for the above-described mesenchymal stem cell markers, and negative for expression of a molecule whose expression is not observed in mesenchymal stem cells. Examples of the molecule whose expression is not observed in mesenchymal stem cells include CD45, CD34, CD14, CD11b, CD79a, CD19, and HLA-DR.

The mesenchymal stem cells according to the present invention have a potential for differentiation into mesodermal cells. Examples of the mesodermal cells into which the mesenchymal stem cells according to the present invention can differentiate include osteoblasts, adipocytes, chondrocytes, osteocytes, cardiomyocytes, tenocytes, dental pulp cells, and odontoblasts, and the mesodermal cells are preferably, for example, osteoblasts, adipocytes, or chondrocytes.

The mesenchymal stem cells acquired by the present invention preferably have a potential for differentiation into one kind or two or more kinds of cells selected from osteoblasts, adipocytes, and chondrocytes, and more preferably have a potential for differentiation into osteoblasts, adipocytes, and chondrocytes.

The mesenchymal stem cells acquired by the present invention can be used in a pharmaceutical raw material, a screening material, and the like in various methods. For example, cells such as osteocytes or adipocytes can be produced from the mesenchymal stem cells using an existing differentiation inducing method. Furthermore, in vitro screening for drug efficacy or toxicity of a drug candidate compound can also be performed using differentiated cells.

In addition, the mesenchymal stem cells obtained by culture from a hair of a donor individual are differentiated into a skin tissue including a hair follicle tissue, and thus the skin tissue or the like can be used for confirmation of the effectiveness of pharmaceuticals, cosmetics, and the like.

### <Method of Producing Differentiated Cells>

Furthermore, the present invention also relates to a method of producing differentiated cells, and the method includes the steps of obtaining a cell population containing mesenchymal stem cells with the method of culturing mesenchymal stem cells of the present invention, and culturing the cell population containing the mesenchymal stem cells in a differentiation induction medium to obtain a cell population containing one kind or two or more kinds of differentiated cells selected from osteoblasts, adipocytes, and chondrocytes.

In a preferred mode, the present invention is a method of producing osteoblasts, and in the method, the differentiation induction medium is an osteoblast induction medium, and the differentiated cells are osteoblasts.

In another preferred mode, the present invention is a method of producing adipocytes, and in the method, the differentiation induction medium is an adipocyte induction medium, and the differentiated cells are adipocytes.

In still another preferred mode, the present invention is a method of producing chondrocytes, and in the method, the differentiation induction medium is a chondrocyte induction medium, and the differentiated cells are chondrocytes.

The osteoblasts according to the present invention include mature osteoblasts, and in addition, immature osteoblasts such as osteoprogenitor cells and preosteoblasts.

Differentiation into the osteoblasts can be confirmed by alizarin red staining. The osteoblasts can be detected by staining calcium deposited on bone-differentiated or calcified cells with alizarin red. Differentiation of the osteoblasts can also be confirmed by alkaline phosphatase (ALP) staining.

The adipocytes according to the present invention include brown adipocytes, white adipocytes, beige adipocytes, and precursors thereof.

Differentiation into the adipocytes can be confirmed by oil red staining. Oil red is a kind of nonpolar and lipid-soluble azo dye, and when in contact with adipocytes, oil red can be dissolved in a solvent of an intracellular lipid to stain a lipid droplet.

Differentiation into the adipocytes can also be confirmed by measuring a triglyceride of the cells.

The chondrocytes according to the present invention include cells that produce an extracellular matrix constituting a cartilage such as collagen, and precursors thereof.

Differentiation into the chondrocytes can be confirmed by Alcian blue staining. Alcian blue ionically binds to a carboxyl group and a sulfate group of an acidic mucopolysaccharide, and thus can stain an acidic mucopolysaccharide such as chondroitin sulfate or hyaluronic acid contained in the chondrocytes.

Furthermore, differentiation into the chondrocytes can be confirmed by confirming the expression of a chondrocyte marker such as SOX9 or ACAN.

Differentiation induction of mesodermal cells can be performed with an existing technique. For example, the differentiation can be induced using a medium in which various differentiation-inducing agents are appropriately added to a known basal medium. Examples of the basal medium include a DMEM medium.

The induction medium for differentiation into the osteoblasts preferably contains one kind or two or more kinds of components selected from ascorbic acid, ascorbic acid-2-phosphate, β-glycerophosphate, dexamethasone, and proteins involved in bone formation (such as bone morphogenic protein (BMP)-2, BMP-4, insulin-like growth factor (IGF)-1, a basic fibroblast growth factor (bFGF), transforming growth factor (TGF)-β1, parathyroid hormone (PTH), and Wnts).

The induction medium for differentiation into the adipocytes preferably contains one kind or two or more kinds of components selected from insulin, dexamethasone, and 3-isobutyl-1-methylxanthine (IBMX).

The induction medium for differentiation into the chondrocytes preferably contains one kind or two or more kinds of components selected from BMP-2, BMP-6, TGF-β3, dexamethasone, ascorbic acid, L-ascorbic acid 2-phosphate, dexamethasone, insulin, an insulin-like growth factor (IGF), L-proline, and a pyruvate.

By the method of producing differentiated cells of the present invention, cells can be obtained that are differentiated into predetermined cells such as osteoblasts, adipocytes, or chondrocytes. That is, the present invention can adopt a mode of differentiated cells (including a cell population) produced with the above-described method of producing differentiated cells. The cells to be acquired may be a cell cluster.

The obtained cells can be further differentiated into various tissues (a bone tissue, a cartilage, an adipose tissue, and the like), and can be suitably used as a material for regenerative medicine.

### Examples

Hereinafter, the present invention will be described in more detail with reference to Examples, but the technical scope of the present invention is not limited to Examples described below.

### <Example 1> Culture of Mesenchymal Stem Cells

### (1) Reagents Used, etc.

### (a) Antibiotic Mix

Into a sterile container, 247 ml of an HBSS was transferred, and 2.5 ml of a penicillin-streptomycin mixed solution (final concentration: 100 U/ml penicillin, 100 mg/ml streptomycin) and 250 µL of an amphotericin B solution (final concentration: 250 ng/ml) were added.

### (b) Medium for Culture of Mesenchymal Stem Cells (DS-MSC Medium)

Into a 50 mL tube, 45 ml of an Essential 8 Flex medium (Gibco, a supplement was already added) was transferred, and 0.5 ml of a penicillin-streptomycin mixed solution (final concentration: 100 U/ml penicillin, 100 µg/ml streptomycin) and 5 mL of FBS were added. Then, a bFGF (REPROCELL Inc.) and an EGF (epidermal growth factor (EGF), human, FUJIFILM Wako Pure Chemical Corporation) were added so that the final concentration of each of the bFGF and the EGF was 10 ng/mL.

### (c) iMatrix Coating

A diluent prepared by adding 5 µL of iMatrix-511 (Nippi, Incorporated) to 1 mL of DPBS (-/-) was added to the bottom surface of a 24-well plate for cell culture in an amount of 300 µL/well, and the plate was allowed to stand in an incubator at 37°C for 30 minutes or more. Then, the diluent was removed, and the plate was used.

### (2) Culture of Mesenchymal Stem Cells

Hairs of a subject (human) were pulled out and immediately immersed into Antibiotic Mix for a moment. Then, hairs having a hair bulb covered with a dermal sheath tissue were selected, and the hairs were cut so as to include a portion to which the hair bulb part and an outer root sheath were adhered. The acquired hairs were each seeded on one well of the iMatrix-coated 24-well plate, to which 150 µL of the DS-MSC medium was added.

One day and 5 days after the seeding, the well was replenished with 150 µL of the DS-MSC medium, and 7 days after the seeding, further replenished with 300 µL of the DS-MSC medium. Subsequently, replenishment and replacement with the DS-MSC medium were continued according to the proliferation level of the cells.

When the cells were proliferated until 50 to 80% of the cells were confluent, passage was performed with the following procedure.

That is, 0.2 mL of TrypLESelect was added, the plate was allowed to stand in an incubator at 37°C, and the cells were detached by pipetting. Then, the cells were collected in a 1.5 mL tube, and after centrifugation, the supernatant was removed, the cells were suspended in 1 mL of the DS-MSC medium, and the number of cells was counted. Then, the cells were seeded on an iMatrix-coated 6-well plate at 50,000 cells/well with the DS-MSC medium.

After seeding the cells, the cells were cultured in an incubator at 37°C. From the day after the seeding, replenishment and total replacement with StemFit for Mesenchymal Stem Cell (Ajinomoto Co., Inc., hereinafter also referred to as StemFit for MSC) were performed, and the mesenchymal stem cells (hair-derived mesenchymal stem cells including a dermal sheath (DS): DS-MSCs) were proliferated.

### (4) Results

Fig. 2 shows the state of cells expanded from the vicinity of a hair bulb 7 days after the seeding on the 24-well plate. As shown in Fig. 2, it was confirmed that a large number of mesenchymal stem cell-like cells were obtained by proliferation from the vicinity of the hair bulb.

Fig. 3 shows the state of cells expanded from the vicinity of an outer root sheath adhered to a hair 6 days after the seeding on the 24-well plate. As shown in Fig. 3, the cells expanded from the vicinity of the outer root sheath adhered to the hair did not show a mesenchymal stem cell-like form, but showed a keratinocyte-like form.

When the passage in (2) described above was performed by mixing cells obtained by proliferation from the vicinity of the hair bulb and cells obtained by proliferation from the vicinity of the outer root sheath adhered to the hair, keratinocyte-like cells obtained by proliferation from the vicinity of the outer root sheath were dead, and only mesenchymal stem cell-like cells were obtained by proliferation.

From the above, it has become clear that according to the method of culturing of the present invention according to Example 1, mesenchymal stem cells can be cultured from a hair including a hair bulb covered with a dermal sheath tissue. Keratinocyte-like cells were obtained by proliferation from the vicinity of the outer root sheath adhered to the hair, but when passage was continued, only mesenchymal stem cells were obtained by proliferation. Thus, it can be said that according to the method of culturing of the present invention, mesenchymal stem cells can be separated and cultured without excising a region including an outer root sheath.

### <Example 2> Differentiation Induction into Osteoblasts, Adipocytes, and Chondrocytes

Differentiation induction into osteoblasts, adipocytes, and chondrocytes was performed on the hair-derived mesenchymal stem cells (DS-MSCs) cultured in Example 1, with the following procedure two days after the third passage.

### (1) Differentiation Induction into Osteoblasts and Adipocytes

In the 24-well plate, it was confirmed that 80 to 90% of the cells became confluent, and the medium was replaced with dedicated media for differentiation induction into osteoblasts and adipocytes. Specifically, MSC Osteogenic Differentiation Medium (PromoCell) was used for inducing osteoblasts, and MSC Adipogenic Differentiation Medium (PromoCell) was used for inducing adipocytes.

After replacing the medium with the differentiation induction media or an undifferentiation maintenance medium (StemFit for MSC) for 2 weeks, alizarin red staining was performed on osteoblasts, and oil red staining was performed on adipocytes. Alizarin red staining was performed with an Alizarin Red S staining kit (Bio Future Technologies) in accordance with an existing protocol. Oil red staining was performed with an Oil Red O staining kit (Bio Future Technologies) using an OR stain diluted to 60% with purified water.

As controls, commercially available adipose tissue-derived mesenchymal stem cells (AD-MSCs, Takara Bio Inc., C-12977, Lot: 458Z017.2), bone marrow-derived mesenchymal stem cells (BM-MSCs, Takara Bio Inc., C-12974, Lot: 479Z025), and umbilical cord-derived mesenchymal stem cells (UC-MSCs, Takara Bio Inc., C-12971, Lot: 458Z025.1) were cultured in a differentiation induction medium or StemFit for MSC in the same manner as the DS-MSCs, and subjected to cell staining.

Next, the appearance of the cells after staining was observed using a light microscope. Fig. 4 shows the results of alizarin red staining, and Fig. 5 shows the results of oil red staining.

### (2) Differentiation Induction into Chondrocytes

In the 24-well plate, it was confirmed that 80 to 90% of the cells became confluent, and then the medium was replaced with a dedicated medium for differentiation induction into chondrocytes (MSC Chondrogenic Differentiation Medium (PromoCell)). After the medium was replaced with the differentiation induction medium for 3 weeks, total RNA was extracted from the cells, and reverse transcription was performed to synthesize cDNA. Using the obtained cDNA, the expression levels of chondrocyte marker genes (SOX9, ACAN) and GAPDH, which is a housekeeping gene, were measured, and the relative expression level of each chondrocyte marker gene with respect to the expression level of GAPDH was analyzed with a quantitative PCR method. Marker gene expression of the BM-MSCs as a positive control was also analyzed. Figs. 6 and 7 show the graphs showing the gene expression levels.

### (3) Results

As shown in Fig. 4, staining with alizarin red was observed in each of the DS-MSCs, the AD-MSCs, the BM-MSCs, and the UC-MSCs cultured using the differentiation induction medium. From these results, it can be said that the mesenchymal stem cells were differentiated into osteoblasts. Note that there was a difference in the proportion of stained cells due to the difference in the origin of the MSCs.

As shown in Fig. 5, staining with oil red was observed in each of the DS-MSCs, the AD-MSCs, the BM-MSCs, and the UC-MSCs cultured using the differentiation induction medium. From these results, it can be said that the mesenchymal stem cells were differentiated into adipocytes. Note that there was a difference in the proportion of stained cells due to the difference in the origin of the MSCs.

As shown in Figs. 6 and 7, in the BM-MSCs cultured using the differentiation induction medium, the expression levels of SOX9 and ACAN were higher than in the case of culture with the undifferentiation maintenance medium. Also in the DS-MSCs cultured using the differentiation induction medium, the expression levels of SOX9 and ACAN were higher than in the case of culture with the undifferentiation maintenance medium.

Therefore, it was confirmed that the DS-MSCs according to the present invention were differentiated into chondrocytes.

From the above, it was shown that the DS-MSCs according to the present invention have a potential for differentiation into osteoblasts, adipocytes, and chondrocytes.

### <Example 3> Confirmation of Mesenchymal Stem Cell Markers

Fluorescence-activated cell sorting (FACS) was performed on the hair-derived mesenchymal stem cells (DS-MSCs) cultured in Example 1, with the following procedure.

The DS-MSCs obtained by three passages were cultured in a T75 flask and proliferated, and the obtained cells were collected by centrifugation. The obtained pellet was suspended in DPBS, and the cells were dispensed into a 1.5 ml tube at 1.0 × 10⁶ cells/mL, and centrifuged to obtain a pellet. To the acquired pellet, 100 µL of 2% FBS/DPBS was added, the pellet was suspended, and the following antibodies were added.

### [Antibodies]

(A) Isotype control CD73 (PE Mouse IgG1 Isotype Ctrl (FC) (Sony Corporation)): 1 µL + Isotype control CD105 (Alexa Fluor 488 Mouse IgG1 Isotype Ctrl (Sony Corporation)): 1 µL
(B) Isotype control CD90 (Brilliant Violet 605 Mouse IgG1 Isotype Ctrl (Sony Corporation)): 2 µL
(C) CD73 (PE Mouse Anti-Human CD73 (BD Biosciences)): 5 µL + CD105 (Alexa Fluor 488 anti-human CD105 (Sony Corporation)): 0.5 µL
(D) CD90 (Brilliant Violet 605 anti-human CD90 (Thy1) (Sony Corporation)): 2 µL

The antibodies and the cells were mixed, shielded from light, and allowed to stand on ice for 30 minutes. The cells were collected by centrifugation, and 500 µL of 2% FBS/PBS was added to suspend and dissolve the pellet. FACS was performed on the obtained sample using CELL SORTER SH800S (Sony Corporation), and the fluorescence was compared between the isotype controls and the target sample.

As shown in Fig. 8, for all of CD105, CD73, and CD90, stronger fluorescence was detected in the target sample than in the isotype controls. Meanwhile, when expressions of negative markers (CD45, CD34, CD14, CD11b, CD79a, CD19, and HLA DR) were confirmed with a similar procedure, fluorescence was not detected for all of the negative markers.

From the above, it was shown that the cells obtained in Example 1 expressed positive markers of mesenchymal stem cells and did not express negative markers.

From the above results, it has become clear that according to the present invention according to Example 1, mesenchymal stem cells can be efficiently cultured from a removed hair. Furthermore, it has been found that hair-derived mesenchymal stem cells including a dermal sheath, obtained according to the present invention, have a potential for differentiation into osteoblasts, adipocytes, and chondrocytes, which are cells into which mesenchymal stem cells are generally said to be capable of differentiating.

### Industrial Applicability

The present invention can be applied to, for example, development of a pharmaceutical preparation using mesenchymal stem cells.

### Reference Signs List

- S1: Acquisition step
- S2: Antiseptic step
- S3: Culture step
- S31: Static culture step

## Claims

1. A method of culturing mesenchymal stem cells, the method comprising a culture step of culturing a hair acquired by removal and including a hair bulb covered with a dermal sheath tissue in a culture solution.

2. The method of culturing according to claim 1,
wherein the mesenchymal stem cells are derived from the hair bulb.

3. The method of culturing according to claim 1,
wherein the culture step includes a static culture step of static-culturing the hair while entire replacement of a medium is not performed and the medium is replenished with a fresh medium.

4. The method of culturing according to claim 3,
wherein a culture period in the static culture step is 6 days or more from a start of the static-culturing.

5. The method of culturing according to claim 1, comprising a step of bringing the hair that is acquired into contact with an antiseptic solution, before the culture step, for less than 1 minute before the culturing.

6. The method of culturing according to claim 1, comprising a step of seeding one of the hair in one well in a culture vessel before the culture step.

7. The method of culturing according to claim 1, not comprising a decomposition step of decomposing a constituent component of an extracellular matrix in the hair before the culture step.

8. The method of culturing according to claim 7,
wherein the decomposition step is a step of treating the hair with a collagenase.

9. The method of culturing according to claim 1,
wherein the mesenchymal stem cells are subjected to adherent culture in the culture step.

10. The method of culturing according to claim 9,
wherein the adherent culture is performed on a culture surface coated with laminin or a fragment of laminin.

11. The method of culturing according to claim 1,
wherein the culture solution contains L-ascorbic acid, selenium, transferrin, and insulin.

12. The method of culturing according to claim 11,
wherein the culture solution contains a basic fibroblast growth factor (bFGF) and/or an epidermal growth factor (EGF) .

13. A method of producing mesenchymal stem cells, the method comprising the method of culturing according to any one of claims 1 to 12.

14. Mesenchymal stem cells that are acquired with the method of culturing according to any one of claims 1 to 12.

15. The mesenchymal stem cells according to claim 14, having a potential for differentiation into one kind or two or more kinds of cells selected from osteoblasts, adipocytes, and chondrocytes.

16. A method of producing differentiated cells, the method comprising the steps of:
obtaining a cell population containing mesenchymal stem cells with the method of culturing according to any one of claims 1 to 12; and
culturing the cell population containing the mesenchymal stem cells in a differentiation induction medium to obtain a cell population containing one kind or two or more kinds of differentiated cells selected from osteoblasts, adipocytes, and chondrocytes.

17. Differentiated cells that are produced with the method of producing according to claim 16.
